(19) Europäisches Patentamt
European Patent Office
Office européen des brevets

(11) EP 0 607 824 B1

(12) EUROPÄISCHE PATENTSCHRIFT

(45) Veröffentlichungstag und Bekanntmachung des Hinweises auf die Patenterteilung:
**22.04.1998 Patentblatt 1998/17**

(51) Int. Cl.[6]: **C07C 17/20**, C07C 51/58, C07C 63/10, C07C 25/13, C07C 63/70, C07C 51/093

(21) Anmeldenummer: **94100203.2**

(22) Anmeldetag: **07.01.1994**

# (54) Verfahren zur Herstellung mehrfach halogenierter Benzotrichloride und Benzoylchloride sowie neue, mehrfach halogenierte Benzotrichloride

Method for the production of multiply halogenated benzotrichlorides and benzoyl chlorides as well as new multiply halogenated benzotrichlorides

Procédé pour la fabrication de trichlorures de benzényles et chlorures de benzoyle multiple halogénés et nouveaux trichlorures de benzényle multiple halogénés

(84) Benannte Vertragsstaaten:
**CH DE ES FR GB IT LI NL**

(30) Priorität: **19.01.1993 DE 4301247**

(43) Veröffentlichungstag der Anmeldung:
**27.07.1994 Patentblatt 1994/30**

(60) Teilanmeldung:
**97103815.3 / 0 781 747**

(73) Patentinhaber: **BAYER AG**
**51368 Leverkusen (DE)**

(72) Erfinder:
- **Marhold, Albrecht, Dr.**
  **D-51373 Leverkusen (DE)**
- **Andres, Peter, Dr.**
  **D-42799 Leichlingen (DE)**

(56) Entgegenhaltungen:
**EP-A- 0 159 388**
**EP-A- 0 393 400**
**EP-A- 0 429 304**
**DE-A- 2 344 603**
**DE-C- 639 578**

- **PATENT ABSTRACTS OF JAPAN vol. 13, no. 187 (C-592) (3535) 2. Mai 1989 & JP-A-01 013 037 (IHARA CHEM.IND.CO.LTD.) 17. Januar 1989**
- **Beilstein Registry Number 1973474**
- **J. Med. Chem., 1992, 35(1), 198-200**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 607 824 B1

Printed by Xerox (UK) Business Services
2.16.1/3.4

## Beschreibung

Die Erfindung betrifft ein Verfahren zur Herstellung mehrfach halogenierter Benzotrichloride II aus den entsprechenden Benzotrifluoriden I in Gegenwart von Chloriden aus der Reihe der Friedel-Crafts-Katalysatoren, ein Verfahren zur Herstellung mehrfach halogenierter Benzoylchloride III aus den Benzotrichloriden II durch partielle Hydrolyse sowie neue, mehrfach halogenierte Benzotrichloride II.

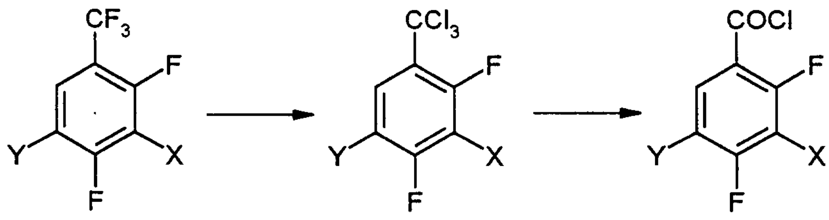

(I) (II) (III)

X = Cl oder F; Y = H oder F.

2,4,5-Trifluor-3-chlorbenzoylchlorid kann nach DE-OS 3 420 796 durch Chlorierung von 2,4,5-Trifluorbenzoesäure mit Chlor und Umsetzung der resultierenden 2,4,5-Trifluor-3-chlorbenzoesäure mit Thionylchlorid erhalten werden.

Aus J. Org. Chem. USSR, 27 (1991)525-532 ist bekannt, daß $CF_3$-Gruppen an Aromaten durch Umsetzung mit Aluminiumchlorid in $CCl_3$-Gruppen überführt werden können.

Es wurde nun ausgehend von den Benzotrifluoriden I ein eleganter Weg gefunden, der über die Benzotrichloride II in guter Ausbeute zu den Benzoylchloriden führt.

Die als Ausgangsprodukte einzusetzenden Benzotrifluoride I lassen sich aus den entsprechenden Tri-/Tetrachlorbenzotrifluoriden durch Chlor/Fluor-Austausch mit Kaliumfluorid herstellen.

Die Menge des einzusetzenden Kaliumfluorids richtet sich nach der Anzahl der auszutauschenden Chloratome. Pro val Chlor wird mindestens ein Mol KF eingesetzt, im allgemeinen jedoch 1,1 bis 1,5 Mol. Maximal werden 2 Mol KF/val Chlor verwendet; darüber hinaus ist die Menge KF praktisch ohne Einfluß auf den Fluorierungsgrad und das Verfahren wird unökonomisch.

Als Lösungsmittel bei der Kernfluorierung können die für Fluorierungsreaktionen bekannten inerten Lösungsmittel, z.B. Dimethylformamid, Dimethylsulfoxid, N-Methylpyrrolidon, Dimethylsulfon, Tetramethylensulfon u.a. verwendet werden. Besonders bevorzugt wird jedoch Tetramethylensulfon (Sulfolan) eingesetzt.

Die Reaktionstemperatur liegt zwischen 160 und 260°C in Abhängigkeit vom gewünschten Fluorierungsgrad. Während bei der niederen Temperatur auch noch kernchlorhaltiges Produkt gefunden wird, entsteht bei den höheren Temperaturen bereits ein recht deutlicher Anteil des bekannten 2,3,4,5-Tetrafluorbenzotrifluorids.

Die oben genannten Tri-/Tetrachlorbenzotrifluoride können durch Chlorieren von beispielsweise 4-Chlorbenzotrifluorid einfach und in hohen Ausbeuten erhalten werden, während dagegen die Chlorierung von Benzoylchlorid oder 4-Chlorbenzoylchlorid zu zahlreichen, die Ausbeute mindernden Nebenprodukten führt.

Gegenstand der Erfindung ist also ein Verfahren zur Herstellung von Verbindungen der Formel II durch Umsetzung von Verbindungen der Formel I mit Chloriden aus der Reihe der Friedel-Crafts-Katalysatoren. Geeignete Chloride dieser Art sind wasserfrei und umfassen beispielsweise Aluminiumchlorid, Titantetrachlorid, Siliciumtetrachlorid, Antimonpentachlorid und Bortrichlorid. Die Menge der Friedel-Crafts-Katalysatoren kann innerhalb weiter Bereiche schwanken; da man jedoch einerseits eine vollständige Umsetzung der Ausgangsverbindungen wünscht und andererseits eine Verschwendung von Chlorid vermeiden möchte, wird man in der Regel 1 bis 2, vorzugsweise 1 bis 1,5 Mol Chlorid pro Mol Benzotrifluorid I einsetzen.

Die Reaktion wird vorzugsweise in einem organischen Lösungsmittel durchgeführt, das unter den Reaktionsbedingungen inert ist, z.B. in Chlorbenzol, in chlorierten oder bromierten $C_1$-$C_4$-Alkanen wie Di- bzw. Tetrachlormethan, Chloroform, Dichlormethan, 1,2-Dichlorethan, Dibrommethan oder in Acetylhalogeniden wie Acetylchlorid oder -bromid. Die Reaktion kann jedoch auch in Abwesenheit organischer Lösungsmittel durchgeführt werden.

Die Reaktionstemperatur kann 0 bis 150, vorzugsweise 10 bis 100°C betragen.

Weiterer Gegenstand der Erfindung sind die Verbindungen der Formel II.

Weiterer Gegenstand der Erfindung ist ein Verfahren zur Herstellung von Verbin-dungen der Formel III durch partielle Hydrolyse von Verbindungen der Formel II mit Wasser in Gegenwart von Eisen(III)-chlorid. Die Wassermenge beträgt im allgemeinen 0,9 bis 1,05 Mol, bezogen auf 1 Mol der zu hydrolysierenden Verbindung II; um eine vollständige Reaktion zu gewährleisten und eine zu weitgehende Hydrolyse zu vermeiden, werden vorzugsweise äquimolare Mengen Wasser eingesetzt. Das Eisen(III)-chlorid kann in Mengen von vorzugsweise 0,1 bis 10, insbesondere 1 bis 3 Gew.-

%, bezogen aufzu hydrolysierende Verbindung II eingesetzt werden. Die Reaktion kann in An- oder Abwesenheit von Lösungsmitteln durchgeführt werden. Als Lösungsmittel eignen sich solche inerten organischen Lösungsmittel, die einen geeigneten Siedepunkt besitzen, wie z.B. Chlorbenzol. Die Reaktionstemperatur kann 80 bis 140°C betragen.

Weiterer Gegenstand der Erfindung ist ein Verfahren zur Herstellung von Verbindungen der Formel III aus Verbindungen der Formel I durch Kombination der oben geschilderten Verfahrensschritte. Dieses Verfahren hat gegenüber der ebenfalls denkbaren Hydrolyse in Verbindungen I zur entsprechenden Benzoesäure und der nachfolgenden Umsetzung zum Benzoylchlorid den Vorteil, daß der Anfall großer Mengen Fluorid enthaltender Schwefelsäure vermieden wird. Das im ersten Teilschritt des erfindungsgemäßen Verfahrens anfallende Aluminiumfluorid ist unlöslich und in der anfallenden Form direkt deponierfähig.

Die erfindungsgemäßen Verbindungen II eignen sich zur Herstellung von Wirkstoffen für Arzneimittel und Futterzusatzstoffe. Beispielsweise kann man aus 3-Chlor-2,4-difluor-benzoylchlorid (Verbindung III; X = Cl, Y = H) 8-Chlor-1-cyclopropyl-7-fluor-1,4-dihydro-4-oxo-3-chinolincarbonsäure nach dem folgenden Mehrstufenverfahren herstellen:

Umsetzung von 3-Chlor-2,4-difluor-benzoylchlorid mit Malonsäurediethylester ergibt (3-Chlor-2,4-difluorbenzoyl)-malonsäurediethylester, partielle Verseifung und Decarboxylierung führt zu (3-Chlor-2,4-difluorbenzoyl)-essigsäureethylester, der mit Orthoameisensäureethylester/Acetanhydrid zu 2-(3-Chlor-2,4-difluorbenzoyl)-3-ethoxy-acrylsäureethylester umgesetzt wird; weitere Umsetzung mit Cyclopropylamin führt zu 2-(3-Chlor-2, 4-difluorbenzoyl)-3-cyclopropylaminoacrylsäureethylester, der mit Kaliumcarbonat/Dimethylformamid zu 8-Chlor-1-cyclopropyl-7-fluor-1,4-dihydro-4-oxo-3-chinolincarbonsäureethylester cyclisiert wird; Verseifung führt dann schließlich zur entsprechenden Carbonsäure. Die beschriebene Reaktionsfolge kann durch das folgende Schema dargestellt werden:

1. Stufe

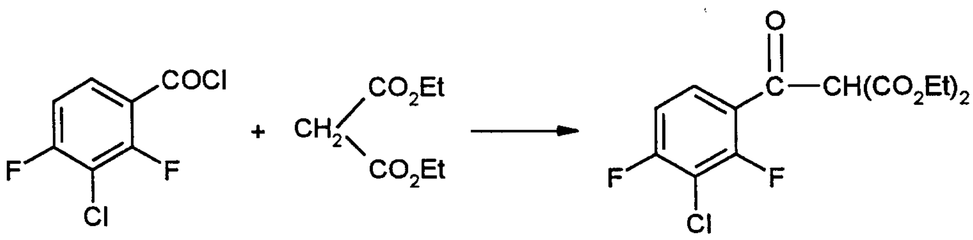

(III) (IV)

2. Stufe

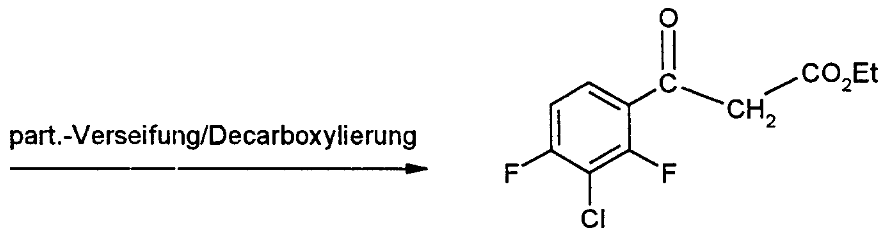


(V)

3. Stufe

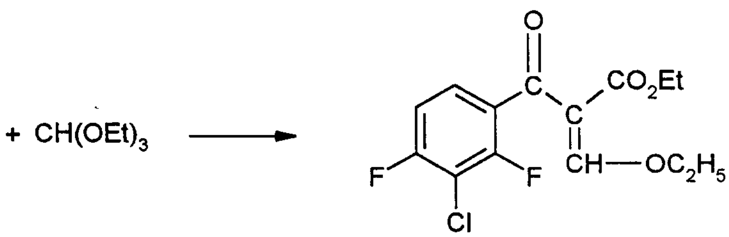


(VI)

4. Stufe

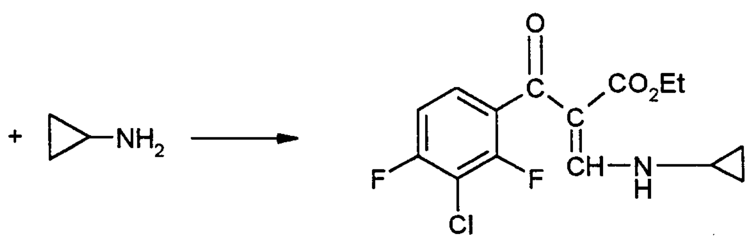


(VII)

5. Stufe

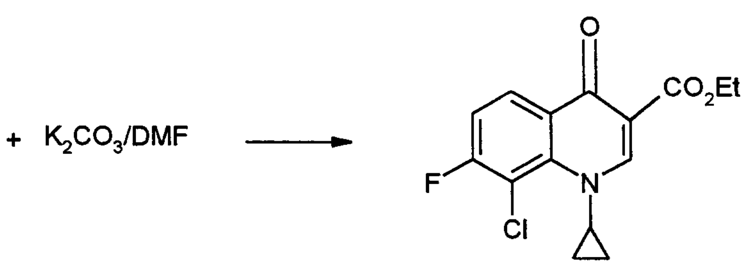


(VIII)

6. Stufe

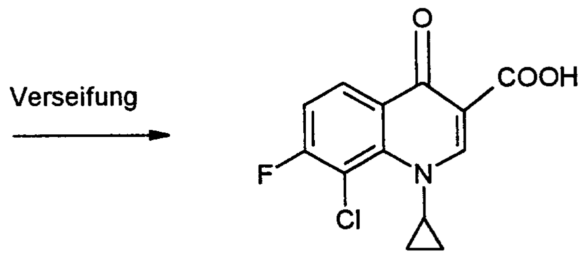


(IX)

Die in der 6. Stufe erhaltene Chinoloncarbonsäure kann durch Umsetzung mit Verbindungen Z-H der Formeln

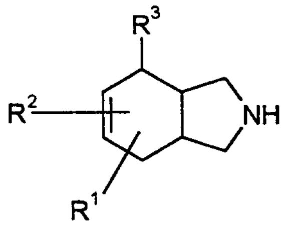

(X)

oder

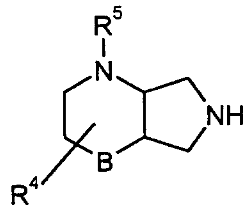

(XI)

,

worin

$R^1$ für Wasserstoff, geradkettiges oder verzweigtes $C_1$-$C_3$-Alkyl oder Cyclopropyl,

$R^2$ für Wasserstoff oder Methyl,

$R^3$ für Wasserstoff, Hydroxy, $-NR^6R^7$, Hydroxymethyl oder $-CH_2-NR^6R^7$,

$R^4$ für Wasserstoff, Methyl oder Reste der Strukturen $-CH=CH-CO_2R'$, $-CH_2CH_2-CO_2R'$, $-CH_2-CO-CH_3$, $-CH_2-CH_2-CN$,

$R^5$ für Wasserstoff oder Methyl,

$R^6$ für Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl,

$R^7$ für Wasserstoff oder Methyl,

R' für Methyl oder Ethyl und

B für $-CH_2-$, -O- oder eine direkte Bindung stehen,

in Chinoloncarbonsäurederivate der Formel

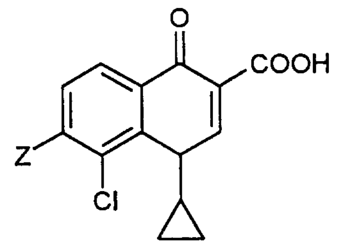

(XII)

überführt werden. Die Verbindungen sind Wirkstoffe mit ausgezeichneter antibakterieller Wirkung.

**Beispiele**

**A. Herstellung der Ausgangsverbindungen**

**3-Chlor-2,4-difluor-benzotrifluorid**

In einer Rührapparatur werden unter Feuchtigkeitsausschluß 3 700 g Kaliumfluorid in 10 000 ml Tetramethylensulfon vorgelegt und bei Druck von 16 mbar 500 ml Tetramethylensulfon abdestilliert. Dann werden 3 965 g 2,3,4-Trichlorbenzotrifluorid zudosiert und auf 230°C erhitzt. Das gebildete Produkt wird über eine Kolonne mit Rückflußteiler abgenommen. Nach drei Stunden wird leichtes Vakuum angelegt und bis zum Siedepunkt des Tetramethylensulfons andestilliert. Das Grobdestillat (3 390 g) wird einer Feindestillation unterworfen. Der Vorlauf von 396 g enthält hauptsächlich 2,3,4-Trifluor-benzotrifluorid (Siedepunkt 92 bis 142°C); der Hauptlauf (3 051 g) hat einen Siedepunkt von 142 bis 143°C und besteht zu 97,6 % aus 3-Chlor-2,4-difluor-benzotrifluorid.

**2,3,4,5-Tetrafluor-benzotrifluorid**

a) Unter Stickstoffatmosphäre (5 bar) wurde eine Mischung aus 852 g 2,3,4,5-Tetrachlor-benzotrifluorid (3 Mol), 1044 g Kaliumflorid (18 Mol), 60 g Kronenether (18 Ringglieder, 6 Sauerstoffatome) und 2050 ml Tetramethylensulfon bei einer Temperatur von 240°C 15 Stunden lang gerührt; während dieser Zeit stieg der Druck auf maximal 12 bar. Unter Normaldruck wurde bis zu einer Innentemperatur von 180°C abdestilliert. Gegen Ende der Destillation wurde Vakuum angelegt; sobald Tetramethylensulfon überging, wurde die Destillation abgebrochen. Das Grobdestillat (485 g) wurde an einer Kolonne redestilliert; man erhielt 387 g 2,3,4,5-Tetrafluor-benzotrifluorid.

b) In einer Rührapparatur aus $V_4$A-Stahl werden 835 g (14,4 Mol) Kaliumfluorid in 1440 ml Tetramethylensulfon vorgelegt und bei einem Druck von 20 mbar Lösungsmittel zur Trocknung abdestilliert (100 ml). Dann werden 852 g (3 mol) 2-H-Tetrachlorbenzotrifluorid und 42 g Tetraphenylphosphonium-bromid zugegeben. Anschließend wird unter Eigendruck (3,5 bar) für 18 Stunden auf 210°C erhitzt. Nach Ende der Reaktion wird langsam entspannt und das Produkt über einen Kühler in eine gekühlte Vorlage entspannt. Zum Schluß wird bei abgesenktem Druck (bis 20 mbar) restliches Produkt bis zum Siedepunkt des Lösungsmittels abdestilliert. Es fallen insgesamt 538 g Destillats an, die nach gaschromatographischer Analyse 407 g ≅ 62,2 % Ausbeute an 2-H-Tetrafluorbenzotrifluorid und 126 g (17,9 % Ausbeute) 5-Chlor-2,3,4-trifluorbenzotrifluorid enthalten.

c) Der Ansatz wird wie unter b) durchgeführt nur mit dem Unterschied, daß vor dem Aufheizen 3 bar Stickstoff aufgedrückt werden und sich ein Gesamtdruck von 6 bar bei 210°C einstellt. Die Aufarbeitung liefert 585 g Grobdestillat, das 400 g (61,1 %) 2-H-Tetrafluorbenzotrifluorid und 170 g 5-Chlor-2,3,4-trifluorbenzotrifluorid enthält. Die Wiederfindungsrate beträgt 85,3 % an fluorierten Benzotrifluoriden.

d) In einer Rührapparatur von $V_4$A-Stahl werden 812 g (14 Mol) Kaliumfluorid in 1400 ml Tetramethylensulfon bei 20 mbar andestilliert (Destillatmenge: 100 ml). Dann werden 40 g Tetraphenylphosphonium-bromid und eine Mischung aus 795 g (2,8 Mol) 2-H-Tetrachlorbenzotrifluorid und 170 g (0,78 Mol) 5-Chlor-2,3,4-trifluorbenzotrifluorid zugegeben und für 18 Stunden unter Eigendruck (max. Druck 3,4 bar) bei 210°C gerührt. Die Destillation liefert 747 g Rohdestillat, das an einer Drehbandkolonne erneut destilliert wird. Es fallen 396 g 2-H-Tetrafluorbenzotrifluord und 318 g 5-Chlor-2,3,4-trifluor-benzotrifluorid an. Die Wiederfindungsrate der isolierten Ausbeute beträgt 90,5 %.

e) Der Ansatz b) wird wiederholt, wobei sonst gleiche Einsatzmengen bei 200°C an Stelle von 210°C und für 24 Stunden (gegenüber 18 Stunden im Ansatz b) gerührt werden. Die destillative Aufarbeitung liefert 596 g Rohdestillat, das 353 g (54 %) 2-H-Tetrafluor-benzotrifluorid und 239 g (34 %) 5-Chlor-2,3,4-trifluorbenzotrifluorid enthält. Die Wiederfindungsrate beträgt 88 %.

**3-Chlor-2,4-difluor-benzotrifluorid**

In einer Rührapparatur werden 800 g Kaliumfluorid und 2500 ml Tetramethylensulfon vorgelegt und bei einem Druck von 15 mbar andestilliert, bis ca. 200 ml Lösungsmittel übergegangen sind. Dann werden bei einer Temperatur von 150°C 1100 g 2,3,4-Trichlor-benzotrifluorid zudosiert und unter Feuchtigkeitsausschluß auf 220°C erhitzt. Das Produkt wird über eine Kolonne mit Rückflußteiler abgenommen; insgesamt wird die Mischung für 12 Stunden bei dieser Temperatur gehalten. Anschließend wird unter vermindertem Druck der Anteil an Fluoraromaten abdestilliert. Nach Redestillation der Reaktionsmischung werden nach einem Vorlauf bestehend aus 2,3,4-Trifluor-benzotrifluorid in einem Siedebereich von 37 bis 40°C/16 mbar 652 g 3-Chlor-2,4-difluor-benzotrifluorid erhalten.

**2,3,4-Trifluor-benzotrifluorid**

Ein HC4-Autoklav wird mit 928 g Kaliumfluorid und 3300 ml N-Methyl-pyrrolidon beschickt und im Vakuum zur Trocknung andestilliert. Anschließend werden unter Feuchtigkeitsausschluß 1732 g 3-Chlor-2,4-difluorbenzotrifluorid zugegeben, 5 bar Stickstoff aufgedrückt und unter Rühren für 10 Stunden auf 270°C erhitzt. Nach Abkühlen wird entspannt und bei leichtem Vakuum bis zum Siedepunkt des NMP destilliert. Die Feindestillation des Rohdestillats ergibt 439 g 2,3,4-Trifluorbenzotrifluorid. Siedepunkt: 104 bis 105°C und 881 g nicht umgesetztes Ausgangsmaterial, das erneut zur Fluorierung eingesetzt werden kann.

**B. Erfindungsgemäße und erfindungsgemäß hergestellte Verbindungen**

**2,3,4,5-Tetrafluor-benzotrichlorid**

85 g (0,64 Mol) Aluminiumchlorid (wasserfrei) werden in 500 ml Dichlormethan vorgelegt und unter Rühren bei Raumtemperatur tropfenweise mit 109 g (0,5 Mol) 2,3,4,5-Tetrafluor-benzotrifluorid versetzt. Dann rührt man 1 Stunde bei 40°C nach, läßt abkühlen, gießt auf 600 g Eis, trennt die organische Phase ab, extrahiert die wäßrige Phase mit Ether, wäscht die vereinigten Phasen mit Wasser und trocknet über wasserfreiem Magnesiumsulfat. Nach dem Einengen wird der Rückstand destilliert. Man erhält 108,3 g (81 % d.Th.) Produkt; Kp: 89 bis 90°C/22 mbar.

**2,3,4,5-Tetrafluor-benzoylchlorid**

In einer Rührapparatur werden 802 g 2,3,4,5-Tetrafluor-benzotrichlorid vorgelegt und 8 g $FeCl_3$ zugegeben. Bei 120°C wird Wasser langsam unter die Oberfläche des Ausgangsmaterials dosiert (insgesamt 54 g Wasser). Es setzt sofort eine starke Chlorwasserstoffentwicklung ein. Der Chlorwasserstoff wird über den Kühler einem Vernichtungsturm zugeführt. Es wird bis Ende der Gasentwicklung gerührt. Anschließend wird das Produkt destilliert. Es werden 569 g 2,3,4,5-Tetrafluor-benzoylchlorid erhalten (89,4 % d.Th.), Kp. 80 bis 82°C/18 mbar.

**2,3,4-Trifluor-benzoylchlorid**

Analog der obigen Vorschrift werden aus 312 g 2,3,4-Trifluor-benzotrichlorid 221 g 2,3,4-Trifluor-benzoylchlorid erhalten; Kp. 78 bis 79°C/15 mbar.

**3-Chlor-2,4,-difluor-benzotrichlorid**

In einer Rührapparatur werden 216,5 g 3-Chlor-2,4-difluor-benzotrifluorid in 440 ml Dichlormethan vorgelegt und 150 g $AlCl_3$ in kleinen Portionen eingetragen. Die Reaktion ist leicht exotherm. Nach Zugabe wird für 2 Stunden auf Rückfluß (42°C) erhitzt, abgekühlt und der Ansatz auf 1l Eiswasser ausgetragen. Nach intensiver Durchmischung wird über eine Nutsche abgesaugt und anschließend die organische Phase abgetrennt. Nach Trocknung wird die Dichlormethan-Phase destilliert. Man erhält 231 g 3-Chlor-2,4-difluor-benzotrichlorid mit einem Siedebereich von 124 bis 126°C/18 mbar.

**3-Chlor-2,4-difluor-benzoylchlorid**

In einer Rührapparatur werden 266 g 3-Chlor-2,4-difluor-benzotrichlorid zusammen mit 4 g $FeCl_3$ vorgelegt. Die Mischung wird auf 110°C erhitzt und langsam 18 g Wasser durch eine Kapillare in den Sumpf dosiert. Der entstehende Chlorwasserstoff wird über einen Intensivkühler einem Vernichter zugeführt. Nach Ende der Zugabe und Gasentwicklung wird abgekühlt und das Rohprodukt destilliert. Im Siedebereich 108 bis 110°C/22 mbar gehen 195 g 3-Chlor-2,4-difluor-benzoylchlorid über ($n_D^{20}$ : 1.5362).

**C. Weiterverarbeitung erfindungsgemäß hergestellter Verbindungen**

**C.1 a) (3-Chlor-2,4-difluorbenzoyl)malonsäurediethylester**

3,9 g (0,16 Mol) Magnesium werden in 8,6 g Ethanol vorgelegt und die Reaktion mit Tetrachlorkohlenstoff gestartet. Bei 50 bis 60°C Innentemperatur wird eine Lösung von 23,1 g (0,144 Mol) Malonsäurediethylester in 16,3 ml Ethanol so zugetropft, daß die Temperatur erhalten wird. Anschließend wird eine Stunde bei 60°C nachgerührt. Danach wird bei -10 bis -5°C eine Lösung von 31,3 g (0,148 Mol) 3-Chlor-2,4-difluorbenzoesäurechlorid in 16 ml Toluol zugetropft, eine Stunde bei 0°C und anschließend unter Erwärmung auf Raumtempertur über Nacht gerührt. Das Reaktionsgemisch wird auf Eiswasser gegeben, mit 10 ml konzentrierter Schwefelsäure angesäuert und mit Toluol extrahiert. Es wird mit gesättigter Natiumchloridlösung gewaschen und das Lösungsmittel im Vakuum entfernt.

Rohausbeute; 49,9 g

**b) (3-Chlor-2,4-difluorbenzoyl)essigsäureethylester**

49,9 g des unter a. erhaltenen Rohproduktes werden in 60 ml Wasser mit 1,83 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.

Rohausbeute: 37,3 g

**c) 2-(3-Chlor-2,4-difluorbenzoyl)-3-ethoxyacrylsäureethylester**

37,3 g des unter b. erhaltenen Rohproduktes werden mit 33,4 g (0,226 Mol) Orthoameisensäureethylester und 37,2 g (0,365 Mol) Essigsäureanhydrid zwei Stunden auf 150 bis 160°C erwärmt. Überschüssiges Reagenz wird zunächst im Vakuum und anschließend im Hochvakuum bis zu einer Badtemperatur von 100°C entfernt.

Rohausbeute: 40,2 g

**d) 2-(3-Chlor-2,4-difluorbenzoyl)-3-cyclopropylaminoacrylsäureethylester**

40,2 g des unter c. erhaltenen Rohproduktes werden in 100 ml Ethanol gelöst und unter Eisbadkühlung 9,6 g (0,168 Mol) Cyclopropylamin zugetropft. Es wird 30 Minuten bei Raumtemperatur nachgerührt und anschließend das Reaktionsgemisch mit 100 ml Eiswasser versetzt. Das ausgefallene Produkt wird isoliert, mit Wasser gewaschen und bei 100°C getrocknet.

Ausbeute. 30,8 g (63 % der Theorie bezogen auf (c))
Schmelzpunkt; 101 bis 104°C

**e) 8-Chlor-1-cyclopropyl-7-fluor-1,4-dihydro-4-oxo-3-chinolincarbonsäureethylester**

15 g (0,046 Mol) des unter d. erhaltenen Rohproduktes werden in 90 ml Dimethylformamid mit 7,2 g (0,052 Mol) Kaliumcarbonat zwei Stunden auf 140 bis 150°C erwärmt. Der erkaltete Ansatz wird auf Wasser gegeben, das Produkt isoliert, mit Wasser nachgewaschen und bei 100°C getrocknet.

Ausbeute: 13,5 g (95 % der Theorie)
Schmelzpunkt: 149 bis 153°C

**f) 8-Chlor-1-cyclopropyl-7-fluor-1,4-dihydro-4-oxo-3-chinolincarbonsäure**

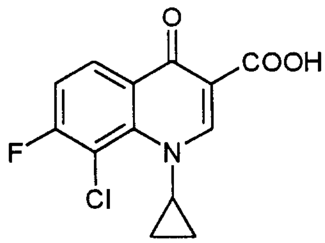

13,5 g (0,044 Mol) des unter e. erhaltenen Esters werden in einer Mischung aus 52 ml Essigsäure, 52 ml Wasser und 5,2 ml konzentrierter Schwefelsäure vier Stunden zum Rückfluß erhitzt. Der erkaltete Ansatz wird auf Eiswasser gegeben, das Produkt wird isoliert, gründlich mit Wasser nachgewschen und bei 100°C getrocknet.

Ausbeute: 11,6 g (94 % der Theorie)
Schmelzpunkt: 192 bis 193°C

**7-(4-Amino-1,3,3a,4,7,7a-hexahydroisoindol-2-yl)-8-chlor-1-cyclopropyl-1,4-dihydro-4-oxo-3-chinolincarbonsäure**

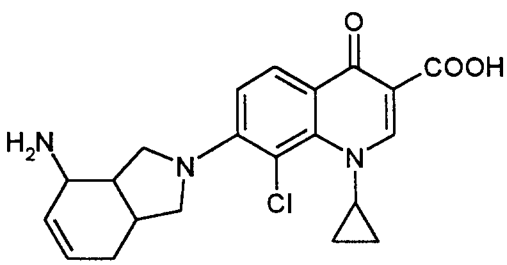

1,26 g (4,5 mMol) 8-Chlor-1-cyclopropyl-7-fluor-1,4-dihydro-4-oxo-3-chinolincarbonsäure werden mit 0,75 g (5,4 mMol) 4-Amino-1,3,3a,4,7,7a-hexahydroisoindol und 1,01 g (9 mMol) 1,4-Diazabicyclo[2.2.2]octan in 45 ml Dimethylsulfoxid eine Stunde auf 100°C erwärmt. Alle flüchtigen Komponenten werden im Hochvakuum entfernt, der Rückstand gründlich mit Acetonitril verrührt und bei ca. 100°C getrocknet.

Ausbeute: 1,7 g (94 % der Theorie)
Schmelzpunkt: 184 bis 186°C (unter Zersetzung)

**C.2 a) (2,3,4-Trifluorbenzoyl)malonsäurediethylester**

3,6 g (0,148 Mol) Magnesiumspäne werden in 8,1 ml Ethanol vorgelegt, die Reaktion mit einigen Tropfen Tetrachlorkohlenstoff gestartet und anschließend die Lösung von 21,8 g (0,136 Mol) Malonsäurediethylester in 15 ml Ethanol und 58 ml Toluol so zugetropft, daß die Innentemperatur zwischen 50 und 60°C liegt. Anschließend wird eine Stunde bei 60°C nachgerührt. Bei -10 bis -5°C wird die Lösung von 27,6 g (0,15 Mol) 2,3,4-Trifluorbenzoesäurechlorid in 15,4 ml Toluol zugetropft, eine Stunde bei 0°C und anschließend unter Erwärmung auf Raumtemperatur über Nacht nachgerührt. Der Ansatz wird auf 60 ml Eiswasser gegeben, mit 9,7 ml konzentrierter Schwefelsäure versetzt und mit Toluol extrahiert. Es wird mit gesättigter Natriumchloridlösung gewaschen und das Lösungsmittel im Vakuum entfernt.

Rohausbeute: 45,2 g

**b) (2,3,4-Trifluorbenzoyl)essigsäureethylester**

45,2 g des bei a. erhaltenen Rohproduktes werden i 57 ml Wasser mit 1,66 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.

Rohausbeute: 33 g

**c) 3-Ethoxy-2-(2,3,4-trifluorbenzoyl)acrylethylester**

33 g des unter b. erhaltenen Produktes werden mit 31,5 g (0,213 Mol) Orthoameisensäureethylester und 31,5 g (0,344 Mol) Essigsäureanhydrid zwei Stunden auf 150 bis 160°C erwärmt. Überschüssiges Reagenz wird zunächst im Vakuum, dann im Hochvakuum bis zu einer Badtemperatur von 100°C entfernt.

Rohausbeute: 34,5 g

**d) 3-Ethylamin-2-(2,3,4-trifluorbenzoyl)acrylsäureethylester**

9,06 g (0,03 Mol) des unter c. erhaltenen Produktes werden in 60 ml Ethanol bei 0°C vorgelegt und 2,12 ml (0,033 Mol) einer 70 %igen Ethylaminlösung zugetropft. Es wird vier Stunden bei Raumtemperatur nachgerührt, 60 ml Wasser zugetropft und das ausfallende Produkt isoliert. Es wird mit Wasser nachgewaschen und bei ca. 100°C getrocknet.

Ausbeute: 5,0 g (55 % der Theorie)
Schmelzpunkt: 106 bis 108°C

**e) 1-Ethyl-7,8-difluor-1,4-dihydro-4-oxo-3-chinolincarbonsäureethylester**

5,0 g (0,017 Mol) des unter d. erhaltenen Produktes werden mit 2,6 g (0,019 Mol) Kaliumcarbonat in 30 ml Dimethylformamid vier Stunden auf 100°C erwärmt. Der erkaltete Ansatz wird auf Eiswasser gegeben, das Produkt isoliert, mit Wasser nachgewaschen und bei 100°C getrocknet.

Ausbeute: 3,6 g (77 % der Theorie)
Schmelzpunkt: 164 bis 166°C

**f) 1-Ethyl-7,8-difluor-1,4-dihydro-4-oxo-3-chinolincarbonsäure**

3,5 g des unter e. erhaltenen Produktes werden in einem Gemisch aus 16 ml Essigsäure, 16 ml Wasser und 1,6 ml konzentrierter Schwefelsäure vier Stunden auf 140°C erwärmt. Der erkaltete Ansatz wird auf Eiswasser gegeben, das ausgefallene Produkt isoliert, mit Wasser nachgewaschen und bei 100°C getrocknet.

Ausbeute: 3,0 g (99 % der Theorie)
Schmelzpunkt: 237 bis 239°C

Das Produkt f) kann mit einem Amin Z-H zum entsprechenden Chinoloncarbonsäurederivat umgesetzt werden.

**Patentansprüche**

1. Verfahren zur Herstellung von Verbindungen der Formel

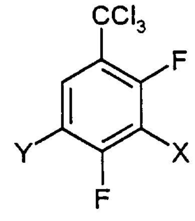

(II) ,

worin

X Cl oder F und
Y H oder F bedeuten,

durch Umsetzung von Verbindungen der Formel

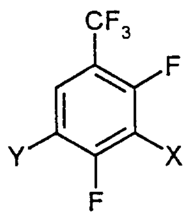

(I) ,

worin X und Y die oben angegebene Bedeutung besitzen,
mit Chloriden aus der Reihe der Friedel-Crafts-Katalysatoren.

**2.** Verfahren nach Anspruch 1, wonach der Katalysator aus der Reihe Aluminiumchlorid, Titantetrachlorid, Siliciumtetrachlorid, Antimonpentachlorid und Bortrichlorid ausgewählt ist.

**3.** Verbindungen der Formel

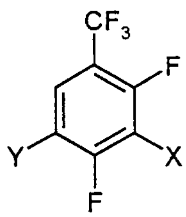

(I) ,

worin

X Cl oder F und
Y H oder F bedeuten.

**4.** Verfahren zur Herstellung von Verbindungen der Formel

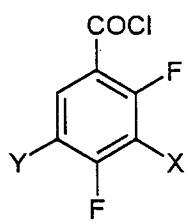

(III) ,

worin X und Y die in Anspruch 1 genannte Bedeutung besitzen,
durch partielle Hydrolyse von Verbindungen nach Anspruch 3 in Gegenwart von Eisen(III)-chlorid.

**5.** Verfahren nach Anspruch 4, wonach man 0,9 bis 1,05 Mol Wasser pro Mol zu hydrolysierender Verbindung des Anspruchs 3 einsetzt.

**6.** Verfahren nach Anspruch 4, wonach man 0,1 bis 10 Gew.-% Eisen(III)-chlorid, bezogen auf zu hydrolysierende Verbindung des Anspruchs 3 einsetzt.

7. Verfahren zur Herstellung von Verbindungen des Anspruchs 4 aus Verbindungen der Formel I, wonach man das Verfahren nach Anspruch 1 und das Verfahren nach Anspruch 4 miteinander kombiniert.

**Claims**

1. Process for the preparation of compounds of the formula

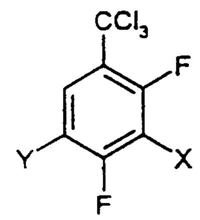

(II) ,

wherein

X denotes Cl or F and
Y denotes H or F,

by reaction of compounds of the formula

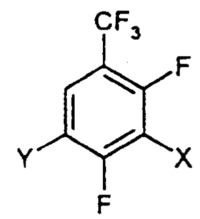

(I) ,

wherein X and Y have the abovementioned meaning, with chlorides from the series of Friedel-Crafts catalysts.

2. Process according to Claim 1, in which the catalyst is chosen from the series aluminium chloride, titanium tetrachloride, silicon tetrachloride, antimony pentachloride and boron trichloride.

3. Compounds of the formula

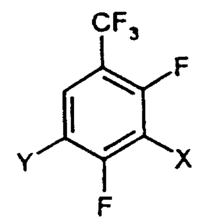

(I) ,

wherein

X denotes Cl or F and
Y denotes H or F.

4. Process for the preparation of compounds of the formula

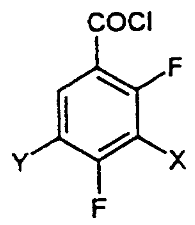

(III) ,

wherein X and Y have the meaning given in Claim 1,
by partial hydrolysis of compounds according to Claim 3 in the presence of iron(III) chloride.

**5.** Process according to Claim 4, in which 0.9 to 1.05 mol of water are employed per mole of compound of Claim 3 to be hydrolysed.

**6.** Process according to Claim 4, in which 0.1 to 10% by weight of iron(III) chloride, based on the compound of Claim 3 to be hydrolysed, is employed.

**7.** Process for the preparation of compounds of Claim 4 from compounds of the formula I, in which the process according to Claim 1 and the process according to Claim 4 are combined with one another.

**Revendications**

**1.** Procédé pour la préparation des composés de formule

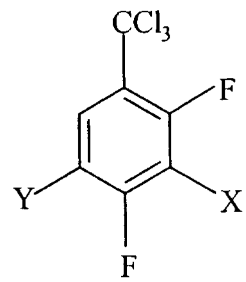

(II),

dans laquelle

X représente Cl ou F et
Y représente H ou F,

par réaction de composés de formule

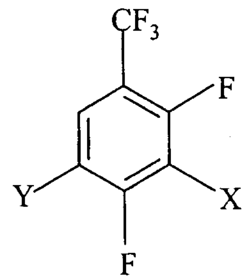

(I),

dans laquelle X et Y ont les significations indiquées ci-dessus,
avec des chlorures de la classe des catalyseurs de Friedel-Crafts.

2. Procédé selon la revendication 1, selon lequel le catalyseur est choisi dans la classe formée par le chlorure d'aluminium, le tétrachlorure de titane, le tétrachlorure de silicium, le pentachlorure d'antimoine et le trichlorure de bore.

3. Composés de formule

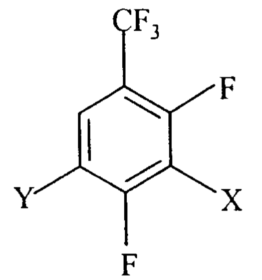

(I),

dans laquelle

X représente Cl ou F et
Y représente H ou F.

4. Procédé de préparation des composés de formule

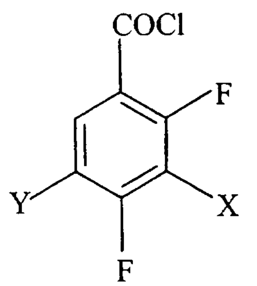

(III),

dans laquelle X et Y ont les significations indiquées dans la revendication 1, par hydrolyse partielle des composés selon la revendication 3 en présence de chlorure de fer-III.

5. Procédé selon la revendication 4, selon lequel on utilise de 0,9 à 1,05 mol d'eau par mol du composé de la revendication 3 soumis à l'hydrolyse.

6. Procédé selon revendication 4, selon lequel on utilise de 0,1 à 10 % en poids de chlorure de fer-III par rapport au composé de la revendication 3 soumis à l'hydrolyse.

7. Procédé de préparation des composés de la revendication 4 à partir des composés de formule I, selon lequel on combine entre eux le procédé de la revendication 1 et le procédé de la revendication 4.